(19) Europäisches Patentamt

European Patent Office

Office européen des brevets

(11) **EP 1 292 362 B1**

(12) **EUROPEAN PATENT SPECIFICATION**

(45) Date of publication and mention
of the grant of the patent:
**04.01.2006 Bulletin 2006/01**

(51) Int Cl.:
***A61N 5/04*** (2006.01)

(21) Application number: **01948516.8**

(86) International application number:
**PCT/US2001/019689**

(22) Date of filing: **20.06.2001**

(87) International publication number:
**WO 2001/098764 (27.12.2001 Gazette 2001/52)**

(54) **SYSTEM FOR HEATING THE PROSTATE GLAND**

SYSTEM ZUR WÄRMEBEHANDLUNG DER PROSTATA

SYSTEME DE CHAUFFAGE DE LA GLANDE PROSTATIQUE POUR TRAITER OU PREVENIR LA
CROISSANCE ET LA PROPAGATION DE TUMEURS DE LA PROSTATE

(84) Designated Contracting States:
**AT BE CH CY DE DK ES FI FR GB GR IE IT LI LU
MC NL PT SE TR**

(30) Priority: **20.06.2000 US 597234**

(43) Date of publication of application:
**19.03.2003 Bulletin 2003/12**

(73) Proprietor: **Celsion Corporation
Columbia, MD 21046-1705 (US)**

(72) Inventors:
 • **FENN, Alan, J.
 Wayland, MA 01778 (US)**

 • **MON, John
 Silver Spring, MD 20905 (US)**

(74) Representative: **Grünecker, Kinkeldey,
Stockmair & Schwanhäusser
Anwaltssozietät
Maximilianstrasse 58
80538 München (DE)**

(56) References cited:
**WO-A-93/08876         WO-A-93/09845
WO-A-99/58194         US-A- 4 813 429
US-A- 5 007 437**

**Description**

**Background of the Invention**

1. Field of the Invention

The present invention generally relates to a system for administering focused energy to a body selectively using either a single or multiple energy applicators applicators in order to treat visable tumors and microscopic malignant and benign cells in prostate tissue with hyperthermia. The system according to the invention may be used to treat healthy tissue containing undetected microscopic pathologically altered cells (neoplasia) that are of high-water content to prevent the occurrence of or the recurrence of cancerous, pre-cancerous or benign prostatic lesions. In addition, the disclosed system can be used to prevent the growth of tumors inside the prostate, as well as prevent the spread of cancer cells outside the prostate.

2. Description of the Prior Art

In order to treat prostate tumors with hyperthermia, it is necessary to heat a significant portion of the prostate gland while sparing healthy tissues in the prostate as well as the surrounding tissues including the urethral and rectal walls of a patient. In the United States there are approximately 200,000 cases of detected prostate cancer annually as well as 375,000 cases of benign prostatic hyperplasia, known as BPH, (enlarged prostate gland). BPH is a non-cancerous enlargement (tumor) of the prostate gland that occurs in almost all men as they age, particularly past the age of 50 years. In the case of BPH, the enlargement of the prostate involves the excessive growth of tissue that eventually obstructs the bladder outlet, creating difficulties with urination. In the case of prostate cancer, eventually the cancer will break through the prostate gland capsule leading to the spread of cancer to the bones and vital organs of the body. Although some of the signs of BPH and prostate cancer are the same, having BPH does not increase the chances of getting prostate cancer. Nevertheless, a patient who has BPH may have undetected prostate cancer at the same time or may develop prostate cancer in the future.

As is known in the art, the use of heat to treat prostate tumors can be effective in a number of ways; however, in most cases, the heat treatment must be capable of heating a significant volume of the prostate gland without overheating the urethral and rectal walls. In radiation therapy, the entire prostate and adjacent tissues are irradiated with x-rays to kill all the microscopic cancer cells. While heating large volumes of the prostate can destroy many or all of the microscopic carcinoma cells in the prostate, known methods of heating tumors can destroy healthy tissue in the prostate and, more damaging, in the urethral and rectal walls of a patient.

The prostate gland has electrical properties similar to muscle (T.S. England and N.A. Sharples, Nature, Vol. 163, March 26, 1949, pp. 487-488.) and is known to have a high-water content, on the order of 80% (F.A. Duck, Physical Properties of Tissue, A Comprehensive Reference Book, Academic Press, New York, p. 321, 1990). Tumor tissue, in general, tends to be 10 to 20% higher in water content than normal tissue (Foster and Schepps, Journal of Microwave Power, vol. 16, number 2, pp. 107-119, 1991). Thus, prostate tumors may have a water content on the order of about 90%. Accordingly, selective microwave heating of the prostate would be the best method of targeting cancerous or benign cells.

It is well known that microwave energy can heat high-water content tumor tissues faster when compared to the heating that occurs in lower-water content normal tissues. Tumor tissue tends to be poorly perfused so blood flow often decreases at therapeutic temperatures allowing rapid heating, while in normal tissues the blood flow often increases protecting the normal healthy tissue from heat damage. Many clinical studies have established that hyperthermia (elevated temperature) induced by electromagnetic energy absorption in the microwave band, significantly enhances the effect of radiation therapy in the treatment of malignant tumors in the human body (Valdagni, et al., International Journal of Radiation Oncology Biology Physics, Vol. 28, pp. 163-169, 1993; Overgaard et al., International Journal of Hyperthermia, Vol. 12, No. 1, pp. 3-20, 1996; Vernon et al., International Journal of Radiation Oncology Biology Physics, Vol. 35, pp. 731-744,1996). Radio-resistant cells such as S-phase cells can be killed directly by elevated temperature (Hall, Radiobiology for the Radiologist, 4th Edition, JB Lippincott Company, Philadelphia, pp. 262-263,1994; Perez and Brady, Principles and Practice of Radiation Oncology, Second Edition, JB Lippincott Company, Philadelphia, pp. 396-397, 1994). Hyperthermia treatments with microwave radiating devices are usually administered in several treatment sessions, in which the malignant tumor is heated to about 43° C for about 60 minutes. It is known that the amount of time to kill tumor cells decreases by a factor of two for each degree increase in temperature above about 43° C (Sapareto, et al., International Journal of Radiation Oncology Biology Physics, Vol. 10, pp. 787-800,1984). Thus, a 60-minute heat-alone treatment at 43° C can be reduced to only about 15 minutes at 45° C, which is often referred to as an equivalent dose ($t_{43°C}$ equivalent minutes).

During treatments with noninvasive microwave applicators, it has proven difficult to heat semi-deep tumors adequately while preventing surrounding superficial healthy tissues from incurring pain or damage due to undesired hot spots. The specific absorption rate (SAR) in tissue is a common parameter used to characterize the heating of tissue. The SAR is proportional to the rise in temperature over a given time interval times the specific heat of the tissue, and for microwave energy the SAR is also proportional to the electric field squared times the tissue electrical conductivity. The units of absolute SAR are watts per kilogram.

The first published report describing a non-adaptive phased array for deep tissue hyperthermia was a theoretical study (von Hippel, et al., Massachusetts Institute of Technology, Laboratory for Insulation Research, Technical Report 13, AD-769 843, pp. 16-19, 1973). U.S. Patent No. 3,895,639 to Rodler describes two-channel and four-channel non-adaptive phased array hyperthermia circuits. Likewise, a non-adaptive phased array hyperthermia system was disclosed in U.S. Patent No. 4,589,423 to Turner.

Bassen et al., Radio Science, Vol. 12, No. 6(5), Nov-Dec 1977, pp. 15-25, shows that an electric-field probe can be used to measure the electric-field pattern in tissue, and in particular, shows several examples in which the measured electric-field has a focal peak in the central tissue. This paper also discusses a concept for real-time measurements of the electric field in living specimens. However, Bassen et al. did not develop the concept of measuring an electric field using real-time with an electric-probe to adaptively focus a phased array.

The most difficult aspect of implementing hyperthermia in deep prostatic tissues, with microwave energy, is producing sufficient heating at a predetermined depth while protecting the urethral and rectal walls and surrounding organs from bums. Noninvasive multiple applicator adaptive microwave phased arrays with invasive and noninvasive electric field probes can be used for producing an adaptively focused beam at the tumor position with adaptive nulls formed in healthy tissues as described in U.S. Pat Nos. 5,251,645, 5,441,532, 5,540,737, and 5,810,888 to Fenn. Ideally, a focused microwave radiation beam is concentrated at the tumor with minimal energy delivered to surrounding healthy tissue. To control the microwave power during treatment, a temperature-sensing feedback probe (Samaras et al., Proceedings of the 2nd International Symposium, Essen, Germany, June 2-4,1977, Urban & Schwarzenberg, Baltimore, 1978, pp. 131-133) is inserted into the tumor, however, it is often difficult to accurately place the probe in the tumor. An additional difficulty occurs in delivering hyperthermia to carcinoma spread throughout the prostate gland, because of a lack of a well-defined target position for the temperature-sensing feedback probe. In other situations, it is desirable simply to avoid inserting probes (either temperature or E-field) into the prostate tissue in order to reduce the risk of infection or spreading the cancer cells when the probe passes through the tumor region.

Several articles have been written on the use of dual intrecavitary (transurethral and transrectal) coherent phased array microwave applicators for prostate cancer treatment (A. Surowiec, et al., Hyperthermic Oncology 1992, Vol. 1, Summary Papers, Proceedings of the 6th International Congress on Hyperthermic Oncology, April 27-May 1, 1992 (Arizona Board of Regents), p. 268 (abstract); M.M. Yeh, et. al. Hyperthermic Oncology 1992, VoL 1, Summary Papers, Proceedings of the 6th International Congress on Hyperthermic Oncology, April 27-May 1,1992 (Arizona Board of Regents), p. 269 (abstract); and J.C. Camart, Hyperthermic Oncology 1996, Vol. 2, Proceedings of the 7th International Congress on Hyperthermic Oncology, Rome, Italy, April 9-13, 1996, pp. 598-600). Further, U.S. Patent No. 5,007,437 to Sterzer describes the use of non-coherent transurethral and transrectal applicators for BPH treatments.

Besides the microwave applicators oriented for irradiating the prostate with microwave energy, means for setting the initial energy power delivered to the applicators, means for monitoring temperatures of walls of the urethra and rectum adjacent the prostate and means for adjusting the relative energy power delivered to the applicators during treatment based on the monitored urethral and rectal wall temperatures are disclosed in WO93/08876.

However, the known prior art is directed to the use of transurethral and transrectal applicators for treating solid tumor masses. None of the known procedures are concerned with treating microscopic disease and preventing the occurrence of solid tumor masses such that occur in cancer and BPH.

*Prostate Cancer*

The current standard of medical care for treating prostate cancer includes radical or nerve-sparing prostatectomy in which the entire prostate gland is surgically removed, and brachytherapy in which radiation seeds at low dose are permanently implanted in the prostate gland radiating effectively for 6 to 9 months or radiation seeds at high dose are temporarily implanted in the prostate for about 2 days, combined with external-beam radiation therapy to catch microscopic cancer cells that may have penetrated or could penetrate the prostate capsule. Side effects from surgery include incontinence and impotence. The cancer recurrence rate after surgery can be as high as approximately 35% at 5 years, and approximately 60% at 10 years, particularly when the prostatic-specific antigen level (discussed below) is greater than 10. Radiation therapy has short-term side effects such as skin reactions, fatigue and nausea. Additional long-term side effects of radiation therapy to the prostate include urinary incontinence (loss of bladder control) and impotence, as well as damage to surrounding organs.

Hormone therapy is also used to supplement prostate cancer treatments by stopping cancer cells from growing. Male hormones, such as testosterone, help cancer cells grow and, in contrast, female hormones or estrogens inhibit growth. Side effects of estrogen therapy include nausea and vomiting, hot flashes, fluid retention, weight gain, headaches and gynecomastia (an increase in breast tissue) in men.

Fundamentally, the problem with current prostate treatments is the inability to control microscopic capsule penetration from the prostate gland, which spreads the cancer to vital organs. Men with microscopic capsule penetration of cancer cells are not cured by radical prostatectomy. These microscopic cells may spread far from the prostate gland into vital organs by means of the lymphatic system or by blood vessels through the prostate capsule.

It is possible to detect the presence of prostate cancer by means of the well-known serum assay Prostate-Specific Antigen (PSA) test (M.K. Brawer, "Prostate-Specific Antigen: Current Status," CA A Cancer Journal for Clinicians, Vol. 49, pp. 264-281, 1999, and J.E. Oesterling, "Prostate Specific Antigen: A Critical Assessment of the Most Useful Tumor Marker for Adenocarcinoma of the Prostate," The Journal of Urology, Vol. 145, pp. 907-923, May 1991.). The prostatic lumen contains the highest concentration of PSA in the human body. PSA is an enzyme produced in all types of prostatic tissue (normal, benign hyperplastic and malignant). In particular, PSA is a serine protease that is produced only by the epithelial cells lining the acini and ducts of the prostate gland; none of the other cellular components of the prostate, including the stromal and vascular elements produce PSA. Researchers have verified that PSA is produced in the epithelial cells of BPH tissue, primary prostate cancer tissue, and metastatic prostate cancer tissue. The serum PSA test detects a significant number of prostate cancers and the destruction of prostatic tumors leads to reduced PSA levels, since the body stops producing PSA when the tumors are eliminated. Currently, a PSA level of 4.0 ng/ml or greater is used to decide whether a patient will be biopsied to try to verify the presence of carcinoma in the prostate. Thus, patients with a PSA level under 4.0 ng/ml currently are not biopsied even if they experience the signs and symptoms of prostate cancer which may include: frequent urination, especially at night, inability to urinate, trouble starting or holding back urination, a weak or interrupted urine flow and frequent pain or stiffness in the lower back, hips or upper thighs.

In addition to PSA level, the Gleason Grade is used to histologically grade adenocarcinoma of the prostate (G.K. Zagars, et al, International Journal of Radiation Oncology Biology Physics, Vol. 31, No. 2, pp. 237-245, 1995), with Grade 1 being the least malignant and slowest growing. Gleason Grade 3 is the most commonly occurring grade when diagnosed. Gleason Grades 4, 5 and above (up to 10) are considered highly aggressive, rapidly growing carcinomas.

Biopsy results and staging are used to predict the behavior of the cancer and the likelihood of its spread. Stage 1 tumors are small and cannot be felt on rectal examination. Stage 2 or greater refers to prostates in which the tumor can be felt. Stage 3 cancers have spread beyond the boarders of the prostate. In Stage 4, which can be determined by imaging studies such as bone scans, CT, or MRI scans, the cancer has spread into nearby lymph glands, the bones, or elsewhere in the body. As is well known in the medical field, the earlier the cancer is detected, the better the chance of being a cancer survivor. If detection is not possible before stage 2, the next best medical option would be to safely treat apparently healthy tissue. Thus, there is a need to treat healthy tissue since cancer, in general, cannot be detected until it has reached stage 2 or a later stage.

There are four types of prostate ductal carcinomas: transitional cell carcinoma, intraductal adenocarcinoma, mixed ductal carcinoma, and endometrioid carcinoma. Transitional cell and mixed ductal carcinomas are aggressive cancers that require complete removal of the prostate and bladder if found while the tumor is still confined to the prostate. Complete removal of the prostate and bladder is also the medically accepted treatment for endometrioid carcinoma. Intraductal adenocarcinomas are treated by radical prostatectomy. Thus, there is a need for a system for treating and preventing the growth and spread of cancer that does not require surgical prostatectomy.

*Benign Prostatic Hyperplasia*

Benign Prostatic Hyperplasia (BPH) is described primarily as an enlargement of the prostate gland that exerts pressure on the urethra, resulting in obstruction of the flow of urine, and is a common affliction in middle-aged and older males. Approximately 50% of men older than 65 years will have BPH symptoms that significantly affect their quality of life. The American Urological Association (AUA) Symptom Index was developed to help categorize BPH symptoms. The AUA score has the following score ranges: 0 to 7 points - BPH symptoms are considered mild; 8 to 19 points - BPH symptoms are considered moderate; and 20 to 35 points - BPH symptoms are considered severe. However, many BPH patients do not seek treatment until their AUA score is about 12.

Over the last two decades, a number of treatments for BPH have been developed each with advantages and disadvantages. The major types of BPH treatment systems are: 1) transurethral resection of the prostate (TURP), 2) Transurethral Electrovaporization of the Prostate (TVP), 3) Drugs, 4) Interstitial Laser Coagulation, 5) RF Needle Ablation, and 6) Microwave Thermotherapy of the Prostate. Other treatment techniques have been explored, including transurethral incision of the prostate, prostatic stents, and balloon dilation, but are used to a lesser extent.

BPH treatment success and practicality can measured in terms of 1) efficacy, 2) durability, 3) level of pain (during and after the procedure), 4) recovery period, 5) complexity of the procedure, 6) cost of the procedure, and 7) side effects. Efficacy of BPH treatments is commonly quantified using the AUA Symptom Index (SI) and peak urine flow rate. Normal urine flow rate is about 16 ml/sec. Other optional tests such as residual urine volume and pressure flow are sometimes used to judge efficacy. Durability is the length of time for which the treatment is effective. The level of pain relates primarily to the need for either general anesthesia or local anesthesia. The recovery period is measured in terms of the number of days of hospitalization and home rest. The complexity of the procedure is a function of the length of the procedure, the framing of the individual administering the procedure (either an urologist or a technician), the type of anesthesia required, and the length of time needed for Foley catheterization after treatment. The cost of the procedure is influenced strongly by the length and complexity of the procedure - particularly whether a hospital stay is required.

Until about 1990, the major treatment ("Gold Standard") for BPH was Transurethral Resection of the Prostate (TURP) which is administered by urologists. TURP is expensive, requires a long recovery time, and has a number of significant side effects, which has prompted the search for better treatment techniques. A summary of approaches to treat BPH, including surgery, drug and laser, RF, and microwave applications is described below.

Transurethral Resection of the Prostate (TURP):

The "Gold Standard" of BPH treatments involves a surgical procedure in which a rigid transurethral scope with an electrosurgical loop is used to remove part of the enlarged prostate tissue (primarily the central zone of the prostate) with RF energy. In practice, 90% of surgical procedures for BPH involve TURP, due to its excellent efficacy (85% or more), long-term durability (10 to 15 years) for 90% of patients. On the order of 200,000 TURPs are performed in the United States annually. TURP has a number of drawbacks: It is a very painful procedure and requires both 2-4 days of hospitalization and 2-4 weeks of recovery at home. The TURP procedure is performed in about one hour and requires general anesthesia. An urologist must perform the procedure. A Foley catheter is required for about 2-3 days post treatment. Some of the major potential side effects of a TURP include impotence, incontinence, high blood loss, and retrograde ejaculation.

Open Prostatectomy:

An open prostatectomy is primarily used on patients with very large prostates with excellent results: the efficacy is greater than 95% and the durability is the same as TURP (10 to 15 years). With any surgical procedure, the pain level is very high and general anesthesia is required. About 7 to 10 days of hospitalization is required with an additional 3-5 weeks spent at home. The procedure takes a few hours and must be performed by a urologist. Following the treatment, a Foley catheter must be used for 2-4 days to drain the bladder. An open prostatectomy is about twice the cost of a TURP, and has the serious potential side effects and complications including high blood loss, impotence, and incontinence.

Transurethral Electrovaporization of the Prostate (TVP):

*Basically a modification of TURP, trausurethral electrovaporization of the prostate* employs a grooved electrosurgical rollerball electrode to channel open the urethra that is blocked by the prostate tissue. The TVP procedure is safer and has minimal side effects compared to TURP. The efficacy is excellent (85%), but still is a very painful procedure requiring 2-4 days of hospitalization and 1-2 weeks at home. A urologist performs this 60-minute procedure and the patient is under a general anesthetic. A Foley catheter must be used for 2-4 days following this procedure. The procedure costs slightly less than TURP. There is less blood loss than with TURP, but their are still the potential side effects of impotence, incontinence, and retrograde ejaculation.

Transurethral Incision of the Prostate (TUIP);

In a relatively new procedure for patients with small prostates, transurethral incision of the prostate provides an efficacy of about 80%. However, TUIP is not effective on large prostates. A minimal amount of prostate tissue is removed in this procedure - a simple incision is made along the entire length of the prostate. The TUIP procedure allows the bladder neck to spring open, allowing free urinary flow. The durability is expected to be similar to TURP, but clinical research is still in progress. This procedure is moderately painful and requires only a day or two of hospitalization, or for some patients is an outpatient procedure. Usually, 4 to 7 days of home rest are needed following the procedure. A urologist must perform this 60-minute surgical procedure and a Foley catheter must be used for 2 to 4 days. The cost of the TUIP is about the same as TURP. There is less blood loss with this procedure compared to TURP, but there are still the potential side effects of impotence, incontinence, and retrograde ejaculation.

Balloon Dilation:

For patients with small prostates, balloon dilation within the prostatic urethra can be used to offer some relief from BPH symptoms. The efficacy is only about 60% and the durability is only 1 to 5 years. This procedure is less costly than TURP and is usually performed as an outpatient with several days of home rest. The procedure is performed under local anesthesia by a urologist in about 30 minutes. A Foley catheter is required for about 2-4 days. There may be some bleeding in this procedure and there are the possible side effects of infection and impotence. The procedure does not work well on large prostates.

Stents:

For very ill patients with small prostates, stents can be used with good effectiveness to improve BPH symptoms. Durability is not a major issue since these patients are usually very ill with other diseases. This procedure is moderately painful and requires only local anesthesia, is performed in about 30-minutes by a urologist, and is done as an outpatient with about 4-5 days of home rest. The cost of this procedure is less costly than TURP. Some of the potential side effects are irritation, infection, and the formation of debris on the stent.

Drugs:

Two categories of drugs are used in treating BPH. One category uses an alpha blocker (Hytrin or Cardura) to relax the muscles that surround the prostate to allow better urinary flow. The other type of drug is reductase inhibitor (Proscar) which actually shrinks the prostate gland.

Hytrin, for example, has very good efficacy (74%) and offers some immediate relief of BPH symptoms, however 2-3 weeks are needed until the full effectiveness is reached. Clinical data suggests that this drug has at least 3 to 5 years durability and is simply prescribed by a general practitioner. The cost is less than TURP depending on the number of years of treatment. There can be some serious side effects such as dizziness, chest pain, irregular heartbeat, and shortness of breath.

Proscar works well on large prostates, but is ineffective on small prostates. Full effectiveness of the drug takes about 3 to 6 months and the durability is estimated at least 3 to 5 years. This drug is prescribed by a general practitioner and must be taken for at least 12 months. The cost of the drug is less than TURP. Some of the known side effects are impotence, swollen lips, decreased volume of ejaculate, and skin rash.

Interstitial Laser Coagulation:

Here, an interstitial laser coagulation surgical device delivers laser energy radially along the length of a custom-designed light diffuser. The diffuser produces an ellipsoidal pattern of thermal damage, applying the laser energy omnidirectionally and uniformly, to maximize treated tissue volume in the prostate. This is a moderately painful surgical procedure, requiring one or two days in the hospital and then 1 to 2 weeks at home. This 30-minute procedure must be performed by a urologist, with a choice of either general or local anesthesia depending on the patient's condition. A serious disadvantage with this procedure is the lengthy required time of 1 to 2 weeks in which a Foley catheter must be used to drain the bladder of urine. The cost of the procedure is less than TURP. There are many potential side effects from this treatment including impotence, incontinence, blood loss, and retrograde ejaculation

RF Needle Ablation (Transurethral Needle Ablation):

This system uses two high-energy RF (approximately 0.47 MHz) needles that are inserted through the urethra into the prostate, to ablate the prostate tissue in a few minutes. More than 10,000 patients worldwide have been treated with this system, which provides good to very good efficacy. There is only limited 12-month durability data for this system, so the long-term effectiveness is not known. The procedure is moderately painful (local anesthesia required) and is performed as an outpatient with 1-2 weeks for home recovery. The procedure is usually performed by a urologist in about 30 minutes. About 40% of patients will require a Foley catheter for about 2 to 3 days. The procedure costs less than TURP. The major side effects from this procedure are irritating voiding, erectile dysfunction, and retrograde ejaculation.

In view of the known treatments for BPH, which require costly, painful, surgery or drugs which have potentially dangerous side effects, there is a need for a system of treating benign prostatic hyperplasia (BPH) that is not painful; can be accomplished on an outpatient basis; and quickly restores the patient to his normal functions. In addition, a method is needed that can safely treat the prostrate gland with focussed energy before a significant amount of microscopic tumor cells form in the prostate.

## Summary of the Invention

The above problems associated with known treatments are solved by the system according to the invention. The system according to the invention safely heats pre-cancerous, cancerous, pre-benign, and benign conditions of the prostate by heating the prostate gland with focussed or concentrated energy, such as microwave energy, delivered by either phase non-coherent or coherent array applicators in the urethra and rectum, or with interstitial applicators positioned within the prostate. In a non-coherent array, separate microwave oscillators can drive the applicators and there is no common phase relation. In a phase coherent array (phased array), a single microwave oscillator can drive multiple applicators with a common phase relation.

The Applicants' approach is to treat the prostate gland with focussed energy, such as microwave energy before a significant amount of microscopic tumor cells form in the prostate gland. As described above, all past uses of thermal therapy were used for the treatment of established prostate cancers with moderate to high PSA levels (over 4.0 ng/ml) or for the treatment of moderate to severe AUA symptom index scores for BPH. The preferred embodiment of this invention is for the prevention or before detection, or before medical intervention is required.

The preferred system comprises coherent adaptive phased array and means for monitoring temperatures of walls of the urethra and rectum, two microwave applicators in at least one of the urethra and rectum, means for adjusting the microwave power to be delivered to the prostate based on the monitored urethral and rectal wall temperatures, means for monitoring the microwave energy dose delivered to the prostate being treated and means for automatically completing the treatment when a desired total microwave energy dose has been delivered by the microwave applicators.

Incoherent-array or non-adaptive phased array hyperthermia treatment systems can be used to heat semi-deep and deep tissue, depending on the radiating frequency. Due to the dielectric heating of high-water content tissue such as prostate tumor, it may be possible to safely heat prostate tumors with either non-coherent arrays or non-adaptive phased arrays.

Moreover, the system according to the invention has application in situations where there is no well-defined position to place the temperature feedback sensor, or where it is desirable to avoid inserting a temperature probe into the prostate tissue. In the case of a single applicator, an E-field probe (or E- field sensors) is not necessary and thus, an invasive probe is not required in the preferred system according to the invention. The inventive system may destroy all of the prostate pre-cancerous and cancerous cells or benign lesions with heat generated by the focussed energy thereby avoiding further progression of the cancer cells or benign lesions.

In addition, the system according to the invention can be used to enhance radiation therapy or for targeted drug delivery and/or targeted gene therapy delivery with or without thermosensitive liposomes as described in U.S. Pat. No. 5,810,888 to Fenn.

The system according to the invention can be used for destroying the pre-cancerous, cancerous, pre-benign, and benign cells in the prostate while preserving normal prostate tissue. Thus, the system according to the invention achieves a thermal prostatectomy and avoids damage to healthy tissue.

The urethral and rectal wall temperature can be measured by temperature probe sensors positioned away from the transurethral and transrectal applicators to obtain the true temperature of the urethral and rectal walls. Alternatively, the tissue temperatures can be monitored by external means, including infrared, laser, ultrasound, electrical impedance tomography, magnetic resonance imaging, and radiometry techniques as known in the art.

Alternatively, a temperature probe could be inserted at an appropriate depth in the prostate tissue to monitor the temperature thereof As discussed below, insertion of a temperature probe is not a preferred embodiment.

In an embodiment with two or more energy applicators, an invasive E-field probe, inserted in the prostate, may or may not be used to measure the microwave power delivered to the tissue to be treated to determine the length of the focussed energy treatment. In a preferred embodiment, the invasive E-field probe can be used to focus the applied energy at the E-field probe inserted in the prostate.

As an alternate embodiment, for a coherent phased array, two E-field sensors can be placed in the prostatic urethra and rectum non-interstitially and be used to null the E-field in the urethra and rectum and effectively focus the microwave radiation in the prostate tissue. Additionally, the microwave phase for the transurethral and transrectal applicators can be adjusted so that the microwave energy is scanned across an area of the prostate.

The system according to the invention can be achieved with or without compression of the prostate. A patient's prostate could be compressed by expanding at least one of a urethral balloon and a rectal balloon. Focussed energy and prostate compression provide preferential heating of high-water content prostate carcinoma and benign cells in the prostate compared to the surrounding lower-water content normal prostate tissues and tissue surrounding the prostate.

To coherently focus the energy, such as microwave energy, in the prostate, the patient's prostate can be compressed via a urethral and rectal balloon and means for determining where to focus the energy in the patient's prostate is used. The means for determining where to focus the energy can be either a single electric-field probe, inserted in the central portion of the prostate, or two non-interstitial electric-field sensors on the urethral and rectal walls. The probe or sensors receive signals that can be used to measure a feedback signal in order to adjust the energy phase delivered to the applicators located in the urethra and in the rectum.

The major advantages offered by treatment according to the system of the invention over known treatments are listed below:

1. Prevention and destruction of prostate tumors (including cancerous and benign);
2. Immediate relief from any BPH symptoms that might exist;
3. Long term durability;
4. Only low level pain may be experienced;
5. Outpatient procedure;
6. Local anesthesia;
7. No Foley catheter required; and
8. No significant side effects or complications.

A biological stent can be formed in the prostatic urethra due to the combination of compression balloon dilation and microwave heat (microwave urethroplasty) as evidenced in clinical tests held by the assignee, Celsion Corporation, during 1999. As a result, one of the major deficiencies in known BPH treatments, namely the need for a Foley catheter for several days, is no longer needed and a patient may experience immediate relief from BPH symptoms.

As described below, applicants' inventive system involves means for monitoring the energy dose delivered to the prostate being treated and means for completing the treatment based on the total energy dose that has been received. Preferably, microwave energy is chosen as the kind of energy the prostate is treated with. That is, conventional temperature feedback measurements of tumor thermal dose can be replaced with the total microwave energy delivered to the coherent phased array or non-coherent microwave applicators and then to the treated area. Accordingly, with the instant invention, instead of temperature feedback measurements, which require the insertion of a temperature feedback probe into the prostate and its inherent problems, microwave energy dose is used as a feedback to determine the required length of treatment. In this application the term "microwave energy dose" (in Joules or watt-seconds) is similar to the dose used in radiation therapy, namely the radiation absorbed dose (Rad) which is a unit of absorbed dose of radiation defined as deposition of 100 ergs of energy per gram of tissue.

Thus, the instant system for selectively heating cancerous and benign conditions of the prostate avoids the risk of spreading cancer cells since the temperature probe is not inserted into the treated area (tumor bed) of the prostate. The elimination of an inserted temperature probe reduces the risk of infection to a patient as a result of the inserted probe. Likewise, the microwave field applied to a tumor would not be subjected to scattering or other disturbance caused by a temperature probe, especially a metallic probe. In addition, the time and costs associated with inserting the temperature probe are saved.

The inventive system may also be used to treat healthy prostate tissue or undetected high-water content microscopic pre-cancerous or pre-benign cells in seemingly healthy prostate tissue to prevent the occurrence of or recurrence of cancerous conditions of the prostate. Thus, the system according to the invention would be able to destroy or ablate microscopic precancerous or pre-benign cells in the prostate gland that are higher in water content (e.g., 90%) than the prostate gland (e.g., 80%) before they are detected. This would be an early treatment that could prevent cancer from growing in the prostate and spreading from the prostate, or enlargement of the prostate gland. In the case of seemingly healthy tissue, the prostate tissue would be irradiated with microwave energy focused at high-water content microscopic cells that are known to form lesions without damaging the healthy lower-water content prostate tissue.

If both transurethral and transrectal applicators are used and both are expanded by respective balloons, a preferred system having means for compressing and immobilizing the prostate to reduce the tissue penetration depth and to reduce the prostate blood flow is achieved.

In an alternate system, the prostate is compressed with a single transurethral balloon, which immobilizes the prostate tissue, reduces blood flow, and reduces the penetration depth required for the microwave radiation. The compression balloon is made of a microwave transparent plastic material such as Latex. The placement of an E-field feedback probe in the prostate may be achieved with an ultrasound transducer or other type of image guidance. Further reduction in blood flow can be achieved, in a preferred method, by injecting a local anaesthetic lidocaine with ephinephrine or anti-angiogenesis drug in the prostate.

Two microwave applicators (such as described by U.S. Pat. No. 5,007,437 to Sterzer) can be positioned transurethrally and transrectally. A phased array can be achieved with a multiple number of applicators greater than or equal to two. In a preferred embodiment, coherent 915 MHz microwave power is delivered to the two transurethral and transrectal applicators, at a predetermined power level, while phase shifters in each channel are adjusted to maximize and focus the microwave energy at the E-field probe sensor. Water-cooling within the catheters and balloons allows cooling of the urethral and rectal walls. Additional interstitial applicators can be inserted within the prostate to supplement the heating that is produced by the transurethral and transrectal applicators.

During the hyperthermia treatment, the microwave power level delivered to each of the applicators may be adjusted either manually or automatically to avoid high temperatures that could cause burns or blisters to the urethral or rectal walls. In addition, the amount of prostate compression, if used, is adjusted as necessary during treatment to provide patient comfort. Each time the prostate compression is adjusted, the microwave-energy/phased array is refocused so that the E-field probe sensor receives maximum power. The total microwave energy, since the start of the treatment, delivered to the microwave applicators is monitored during the treatment. The treatment is completed when a desired amount of total microwave energy is delivered to the microwave applicators, which indicates that the lesion cells are significantly destroyed (i.e., thermal downsizing) or completely destroyed (i.e., thermal prostatectomy).

In order to determine the effectiveness of the treatment, the prostate tissue may be imaged and examined with one of x-ray, ultrasound, and magnetic resonance imaging before and after the microwave total energy dose is administered, as well as with pathological results from needle biopsy of the prostate tissues.

In an alternate embodiment of the invention, the single invasive E-field probe is replaced with two E-field sensors positioned in the urethra and rectum and the coherent array is phase focused by minimizing (nulling) the individual or combined power received by the two sensors, providing a completely noninvasive treatment. In a preferred embodiment, the two E-field sensors are contained with catheters attached to the outside surface of a compression balloon which provides a pressure contact to the urethal and rectal walls. Algorithms are used in conjunction with the feedback signals sensed by the E-field sensors to null areas on the urethral and rectal walls outside thereby focussing the applied energy on an internal site. After the nulling algorithm is completed, the E-field sensors can be withdrawn and temperature sensors can be inserted to measure the urethal and rectal wall temperatures.

Such a totally non interstitial hyperthermia treatment where E-field sensors and temperature sensors monitor the urethral and rectal walls would provide an effective method of destroying benign and cancerous lesions in the prostate. In an embodiment with non-coherent applicators, an E-field focussing probe and phase shifters are not required to heat the tissue. With non-coherent energy, only the applicator radiated power is additive and phase shift is not used.

While the preferred embodiment is described with reference to adaptive microwave phased array technology, Applicants' system may be achieved by focussing energy, in general, to heat and ablate an area of tissue. The focused energy may include electromagnetic waves, ultrasound waves or waves at radio frequency That is, applicants' inventive system includes any energy that can be focused to heat and ablate an area of tissue. This energy, such as microwave or ultrasound energy, can be coherent or non-coherent

In yet another embodiment of the invention with a coherent phased array, the boundary of an area of tissue to be treated in a body (e.g., prostate) is calculated, an E-field probe may be inserted in the body or at least two E-field sensors are positioned within the urethra and rectum; and energy is applied through applicators to the area to be treated. In this embodiment, the focus of the energy would change so that the focus scans the area to be treated. That is, there is no longer a fixed focus spot as the relative phase of the applied energy would be adjusted so that the focus moves inside the area to be treated thereby obtaining a geometric shape of heating.

A fixed focus spot is determined through the appropriate algorithm. Then, for example, the relative phase of the applicators to obtain this fixed focus spot is adjusted 30° one way and then 30° the other way to "scan" a larger heated/treated area. Depending on the size of the area to be treated the scan may focus between 180° and 90° or 60° or 120°.

Further objectives and advantages will become apparent from a consideration of the description and drawings.

**Brief Description of the Drawings**

The invention is better understood by reading the following detailed description with reference to the accompanying figures, in which like reference numerals refer to like elements throughout, and in which:

Fig. 1 shows the microwave thermotherapy system according to a preferred embodiment of the invention for heating the prostate under compression from coherent transurethral and transrectal applicators; and
Fig. 2 shows the microwave thermotherapy system according to a preferred embodiment of the invention for heating the prostate under compression from non-coherent transurethral and transrectal applicators.

**Detailed Description of the Preferred Embodiment**

Description of the Prostate Gland and its Microwave Properties

The prostate gland 220 is part of the male reproductive system and is a solid, walnut-shaped organ that surrounds the first part of the urethra 205 immediately under the bladder 202 and in front of the rectum 210. Prostate cancer arises from the glands of the prostate and the most common form of prostate cancer is known as adenocarcinoma, which means a cancer of the glands. Most prostate cancers develop within the bottom portion of the prostate (sometimes referred to as the peripheral zone which involves roughly 70% of the glandular prostate) closest to the rectum, and this is the region that needs a significant amount of treatment. While a digital rectal exam is useful in detecting hardened areas or lumps in the prostate gland, however it is not very useful for detecting microscopic prostate disease. The use of a transrectal applicator (in addition to a transurethral applicator) to reach this portion of the prostate is essential for a complete treatment of the prostate. The central zone of the prostate (closest to the bladder) is relatively immune to both BPH and prostate cancer diseases. BPH arises mainly in the transition zone located between the central zone and peripheral zone.

As discussed above, current medical procedure does not biopsy a tumor until a PSA of 4.0 ng/ml is reached. The data shown in Table 1 indicate that there is only about a 15% probability of detecting cancer by needle biopsy when the PSA is less than 4 ng/ml. Although the probability of detecting the cancer is very low, the actual probability that there are microscopic cancer cells in the prostate is significant (25% or more) (F.H. Schröder et al, The Journal of Urology, Vol. 163, No. 3, p. 806 (abstract), March 2000) (Eschenbach et al., CA Cancer J. Clinicians, Vol. 47, pp. 261-264, 1997). Thus, thermotherapy treatment of the prostate

Table 1. Probability of detecting cancer on initial biopsy for different levels of PSA. is likely warranted, even if the PSA level is in the range of 0 to 4 ng/ml. Thermotherapy treatment of the prostate for PSA levels below 4 ng/ml is intended to kill the microscopic cancer cells in the prostate and keep the PSA from rising above 4 ng/ml.

| PSA Level | Probability of Detecting Prostate Cancer on Initial Biopsy |
|---|---|
| 2 ng/ml | 1% |
| 2-4 ng/ml | 15% |
| 4-10 ng/ml | 25% |
| > 10 ng/ml | >50% |

Microwave radiation in the Industrial, Scientific, Medical (ISM) band 902 to 928 MHz is commonly used in commercial clinical hyperthermia systems, and is the primary frequency band considered here. It is known that the prostate is high-water content tissue and, hence, is similar to muscle tissue which is well characterized. For normal prostate tissue at 915 MHz, the average dielectric constant is 50 and the average conductivity is 1.3 S/m. The calculated loss due to attenuation of a 915 MHz plane wave propagating through prostate tissue is approximately 3 dB per cm. Prostatic intraepithelial neoplasia, also known as atypical hyperplasia and intraductal dysplasia are pre-cancers and are associated with the development of adenocarcinoma of the prostate. The neoplastic cells are assumed to be higher-water content than the surrounding normal prostate cells and will be heated faster than the normal healthy prostate cells. The normal ductal tissue in the prostate is assumed to be in the low- to medium-water content range.

The safety of employing radiofrequency (microwave) electromagnetic fields in order to treat cancer has been questioned. A comprehensive study recently concluded that there is no association between the incidence or promotion of cancer and exposure to radiofrequency electromagnetic fields in the frequency range of 3 KHz to 300 GHz (L.N. Heynick, Radiofrequency Electromagnetic Fields (RFEMF) and Cancer: A Comprehensive Review of the Literature Pertinent to Air Force Operations, AFRL-HE-BR-TR-1999-0145, United States Air Force Research Laboratory, Directed Energy Bioeffects Division, June 1999). Thus, based on this report, the Applicants realized that there is significant evidence that microwave treatment of the prostate can safely heat an apparently healthy prostate gland containing microscopic cancer cells such that no new cancer would be formed as a result of the microwave treatment.

System for Heating Prostate Tissues

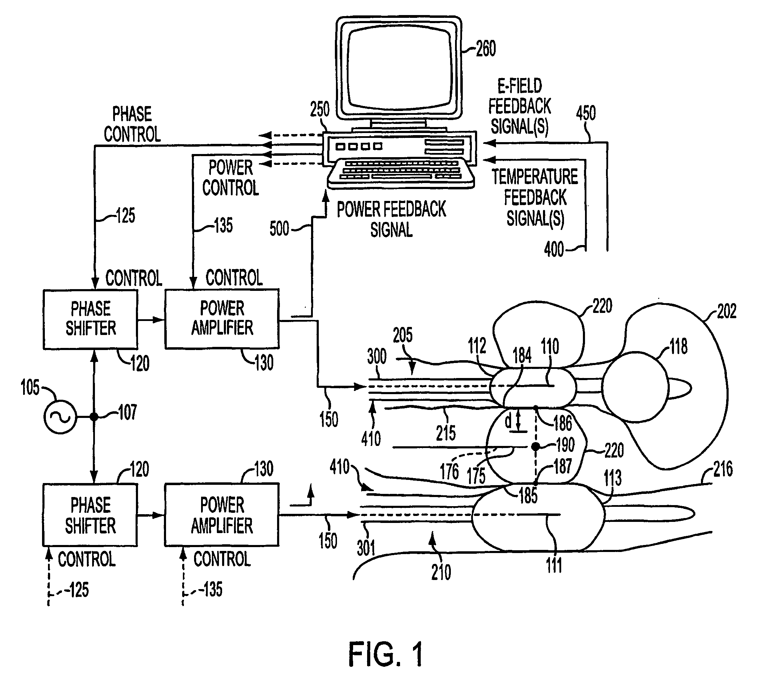

Figure 1 shows a preferred system for heating carcinomas and benign tumor cells in prostate tissues, using an adaptive energy, preferably microwave, phased array hyperthermia system with E-field and temperature feedback. In order to heat deep tissues reliably at energy frequencies, it is necessary to surround the body (prostate lobes **220)** with two or more energy applicators **110, 111** (within the urethra **205** and rectum **210,** respectively) controlled by an adaptive phased array algorithm. The energy applicators **110, 111** may be coherent microwave applicators. The blackened circle, indicated as focus **190,** represents a central tumor or healthy tissue of the prostate **226** that is to be treated.

Focus **190** may represent cancerous conditions of the prostate including one of adenocarcinoma, carcinosarcoma, rhabdomyosarcoma, chondrosarcoma, and osteosarcoma, or pre-cancerous conditions including one of prostatic intraepithelial neoplasia, and benign prostate lesions including benign prostatic hyperplasia. In addition, the system according to the invention, can treat apparently healthy tissue in order to prevent the occurrence or re-occurrence of cancerous or benign conditions.

In the preferred embodiment, an E-field feedback probe **175** can be inserted to an appropriate depth in the tissue of prostate **220** that is to be treated. Insertion of E-field feedback probe **175** may be achieved under the guidance of an ultrasound transducer. Means for setting the initial energy phase delivered to each applicator **110, 111** includes E-field feedback signals **450** from the E-field probe **175** and computer **250** with an appropriate algorithm in order to focus the energy radiation at the inserted E-field probe **175**. Preferably, the E-field probe **175** is used with an adaptive phased array fast-acceleration gradient search algorithm, as disclosed in U.S. Pat. No. 5,810,888 to Fenn, to target the energy radiation at the tumor site **190**.

In addition, the system according to the invention includes means for setting the initial energy or microwave power delivered to each energy applicator, and means for monitoring the temperatures of walls of the urethra and rectum adj acent the prostate that is to be treated to ensure that those walls are not overheated. The means for monitoring urethral and rectal walls may include temperature feedback sensors **410** that are inserted non-interstitially against the urethral and rectal walls **(215, 216)** in order to monitor the temperatures of the urethral and rectum walls adjacent the prostate tissue. Temperature feedback sensors **410** send temperature feedback signals **400** to computer **250** where signals **400** are used to adjust the relative microwave power level that is to be delivered to applicators **110, 111** to heat the tumor or tissue at focus **190**.

Preferably, the design of the transurethral and transrectal energy applicators, which preferably are microwave applicators, is according to U.S. Pat No. 5,007,437 to Sterzer. The transrectal applicator, in particular, may use a reflector or a phased array to direct microwave energy preferentially towards the prostate. The applicators can be noninvasive applicators such as waveguide, monopole, or dipole antennas, or interstitial applicators such as monopole or dipole antennas. In a preferred embodiment, the applicators can be driven coherently as a phased array. In addition, multiple applicators surrounding the prostate that can be driven non-coherently in a multiple frequency array can be used to selectively heat the prostate tissue.

Preferably, the body or prostate **220** is compressed between two compression balloons **112,113,** which surround transurethral and transrectal applicators **110, 111,** respectively. Compression balloons **112, 113** can be inflated with distilled or deionized water. In the alternative, the compression balloons **112, 113** can be inflated pneumatically or by other known means to inflate balloons. Compression balloons **112, 113** are made from a material such as latex that is transparent to microwaves. In addition to immobilizing the prostate tissue and fixing the positions of the applicators, prostate compression has a number of potential advantages for hyperthermia treatments. Utilization of prostate compression results in less penetration depth required to achieve deep microwave heating and reduces blood flow, which also improves the ability to heat tissue.

Compressing the prostate from the inside and outside of the prostate moves the surface of the prostate gland farther from the microwave applicator radiators, which helps to reduce superficial hot spots. In a preferred embodiment, the applicator would have a fluid filled cavity that would improve coupling of the microwave energy from the applicator to tissue to be treated. Cooling of the fluid, such as distilled or deionized water, within the transurethral and transrectal applicators or applicator balloons during hyperthermia treatments helps avoid the potential for developing hot spots in the urethra **205** and rectum **210** thereby protecting the urethral and rectal walls from overheating.

Prior to the adaptive phased array hyperthermia treatment, the prostate is compressed between compression balloons **112,113** and a single invasive E-field feedback probe **175** is inserted within the central tissue site (focus **190**) in the prostate, parallel to the polarization of the microwave applicators **110, 111**. The microwave applicators **110, 111** are either monopole or dipole antenna radiators of straight or helical shape. E-field probe **175** is used to monitor the focal E-field amplitude as the phase shifters are adjusted for maximum feedback signal using an adaptive phased array gradient search algorithm. Noninvasive temperature sensors **410** monitor the urethral and rectal wall temperatures at positions **184,185,** respectively, and these signals are individually transmitted to the computer as temperature feedback signals **400**.

The tips of temperature sensors **410** can be attached to the outside of the transurethral and transrectal compression balloons **112,113** as long as the tips are thermally insulated. The thermal isolation can be achieved by mounting a thin pad (not shown) between the temperature probe and the outside surface of the balloon, from the effects of the cooling fluid contained with the compression balloons. The dual-applicator adaptive phased array of the invention together with the E-field feedback probe allows the phase shifters to be adjusted so that a concentrated E-field can be generated permitting focused heating in tissue at the appropriate depth.

Preferably, temperature sensors **410** are non-interstitially inserted through the openings of the urethra **205** and rectum **210** so that the sensors **410** are in pressure contact with the respective, urethral and rectal wall. Thus, as shown in Figure 1, two temperature feedback probe sensors **410** are located in the urethra **205** and rectum **210,** respectively and produce temperature feedback signals **400**. Two microwave water-cooled catheters **300, 301** with microwave applicators **110, 111,** respectively, are positioned in the urethra **205** and rectum **210**. Transurethral catheter **300** contains a Foley balloon **118** that is air inflated in the bladder **202** to fix the microwave applicator **110** in the correct position with respect to the target area of the prostate.

For coherent treatments, an oscillator **105** is divided at node **107** and feeds phase shifters **120**. Oscillator **105** in a preferred method is a microwave energy source at approximately 915 MHz. The phase control signal **125** controls the phase of the microwave signal over the range of 0 to 360 electrical degrees. The microwave signal from each phase shifter **120** feeds into microwave power amplifiers **130**. The resultant microwave signal is controlled by a computer-generated control signal **135,** which sets the initial microwave power level delivered to each microwave applicator. Microwave signals **150** in the form of coherent 915 MHz microwave power is delivered by the microwave power amplifier **130** to the two applicators **110,111** while phase shifters **120** in each channel are adjusted to maximize and focus the microwave energy at the E-field probe sensor **175** so that microwave power is maximized at the focus position **190**. The treatment then begins.

In another embodiment, the means for monitoring temperature of the prostate is a temperature probe that is inserted at an appropriate depth in the prostate tissue. In this case, after the means for setting the initial relative energy phase delivered to each applicator is focussed at the E-field probe **175**, the E-field probe can be removed and the temperature probe **176** can be inserted in its place temperature at an appropriate depth in the prostate tissue.

In yet another embodiment envisioned by the invention, a second temperature monitoring means, in addition to the non-interstitial temperature sensors in pressure contact with the walls of the urethra and the rectum, is provided. The second temperature monitoring means is the invasive temperature probe 176 that is inserted in the prostate tissue in the same spot from which the E-field probe **175** removed.

The system according to the invention enables all of the treated prostate carcinomas, pre-cancerous cells, and benign lesions to be destroyed when the desired total microwave energy dose has been delivered to the microwave applicators while avoiding damage to the normal tissue of the prostate.

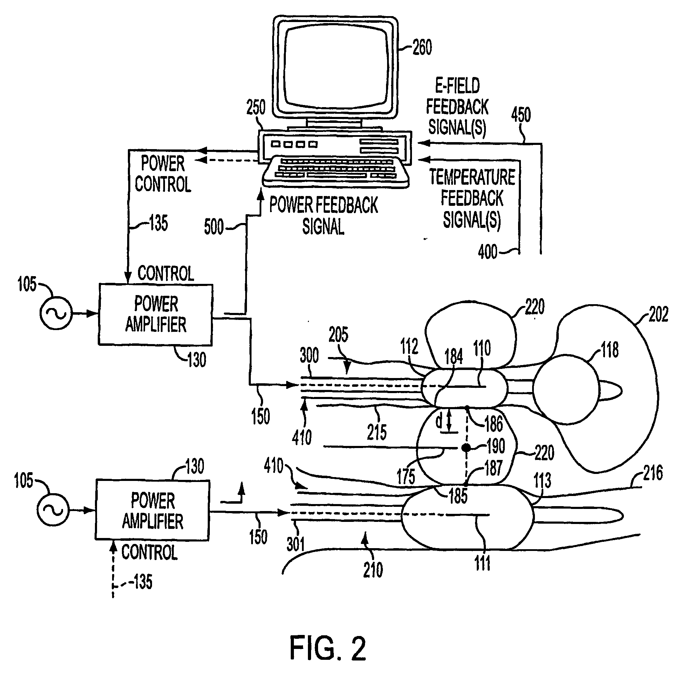

For non-coherent treatments, as shown in Figure 2, separate oscillators **105,** which preferably operate at 915 MHz, feed the two microwave power amplifiers 130 that are computer controlled and deliver microwave power to the two applicators **110, 111.**

During the hyperthermia treatment, the microwave signals **150** and power level delivered to each of the applicators is measured as a power feedback signal **500,** which is sent to a microprocessor or computer **250** such as a PC. The power control signal of the power amplifiers **130** is adjusted either manually or automatically to control the urethra and rectum temperatures, as well as, the equivalent thermal dose delivered to the prostate tissue. The sensors **410** measure the urethral and rectal wall temperatures and the power control signal **135** is adjusted based on the sensed temperature to avoid high temperatures that could cause burns or blisters. The amount of compression realized by compression balloons **112, 113** is adjusted as necessary during treatment to provide patient comfort. Each time the prostate compression is adjusted, for coherent treatments, the phase shifters **120** are readjusted/refocused so that the E-field probe 175 receives maximum power.

According to the system according to the invention, means for monitoring the microwave energy delivered to the microwave applicators **110,111** monitor the delivered energy/power during treatment and when the desired total microwave energy has been delivered by the microwave applicators **110,111** to the prostate, means for terminating the treatment turn off the energy radiation to the applicators. That is, the system according to the invention automatically turns off the energy that is being delivered to the prostate, thereby completing the hyperthermia treatment when a desired total microwave energy dose has been delivered to the prostate **220**. In a preferred embodiment, the total microwave energy dose produces a total equivalent thermal dose in prostate tumors, which is approximately between 60 minutes and 400 minutes relative to 43 degrees Celsius. The total microwave energy, since the start of the treatment, delivered to the microwave applicators is computed within computer **250** and can be displayed on the computer monitor **260** during the treatment.

As an alternate embodiment, means are provided for monitoring the microwave power level delivered to the E-field probe **175** to determine when the treatment should be terminated. According to this embodiment, the total microwave energy calculated from the E-field feedback signal **450** received by the E-field probe **175** is used to control the length of the treatment. This E-field feedback signal **450** can be useful for both coherent and non-coherent treatments. In order to determine the effectiveness of the treatment, the prostate tissue is imaged with one of x-ray and magnetic resonance imaging before and after the microwave total energy dose is administered, as well as pathological results from needle biopsy of the prostate tissues.

For coherent treatments, the single invasive E-field probe **175** can be replaced with two noninvasive E-field sensors at fixed positions **186, 187** within the urethra and rectum, respectively. The E-field sensors are inserted in the natural openings of the urethra **205,** and rectum **210** and fixed in a position that is suitable to heat the tumor or healthy tissue. E-field sensors can be attached to the urethral and rectal walls at positions **186,187,** but they do not have to be in contact with the urethral and rectal walls. Ultrasound, x-rays or other known E-field monitoring device can verify the suitable E-field sensor positions. The total power measured by the two noninvasive E-field sensors is minimized (as in U.S. Pat. No. 5,810,888) by adjusting the microwave phase shifters 120, to create a focused E-field in the central portion of the prostate, or the area of the prostate to be treated.

With this embodiment, there is no risk of infection due to an inserted probe, no risk of scarring of the skin by a procedure which requires nicking the skin and inserting a probe, and no risk of spreading cancer cells as an inserted E-field probe is not used. The E-field sensors merely are fixed within the urethra and rectum and thus, do not pass through a tumor bed thereby reducing the possibility of inadvertently seeding viable cancer cells during a surgical procedure, thus reducing local recurrences of the cancer in surrounding tissues. Likewise, since both the temperature and E-field sensors can be placed in the urethra 205 and rectum 210 according to this method, the instant invention would work well when there is no defined single area in the prostate such as in the treatment of microscopic prostate disease.

Preferably, each channel (on either side of node **107**) of the phased array contains an electronically-variable microwave power amplifier **130** (0 to 100 W), an electronically-variable phase shifter **120** (0 to 360 degrees), and water-cooled microwave applicators **110, 111.**

While the preferred embodiment discloses microwave energy at approximately 915 MHz, the frequency of the microwave energy may be between 100 MHz and 10 GHz. The frequency of the microwave energy could be selected from the range of 902 MHz and 928 MHz. In fact, lower frequencies of energy may be used to ablate or prevent cancerous tissue.

In a preferred embodiment, the initial microwave power delivered to each applicator is between 0 and 70 Watts, preferably between 20 and 60 Watts. Over the entire treatment of the tissue, the microwave power delivered to each applicator may be adjusted over the range of 0-150 Watts to deliver the desired microwave energy dose and to avoid overheating the urethra and rectum. In addition, the relative microwave power delivered to the two microwave applicators is adjusted between -180 degrees and 180 degrees before and during the treatment to create a focussed field in the prostate tissue. Typically, more microwave power is required for non-coherent applicator treatments than for coherent applicator treatments.

In a preferred embodiment, a 0.9-mm outside-diameter (OD) invasive E-field coaxial monopole probe (semi-rigid RG-034), with the center conductor extended 1 cm, can be used as E-field probe **175** to measure the amplitude of the electric field directed to the tissue and to provide the feedback signal **450** used to determine the necessary relative phase for the electronic phase shifters prior to treatment. Coaxially-fed monopole probes of this type have been used to make accurate measurements of linearly polarized electric fields in compressed breast phantoms (Fenn et al., International Symposium on Electromagnetic Compatibility 17-19 May 1994 pp. 566-569). This linearly-polarized E-field probe is inserted within a 1.6 mm OD teflon catheter. Thermocouple probes (Physitemp Instruments, Inc., Type T copper-constantan, enclosed within a 0.6 mm OD teflon sheath) can be used to measure the local temperature in the tumor during treatment. These temperature probes have a response time of 100 ms with an accuracy of 0.1° C. Fiber-optic temperature probes may also be used.

The E-field probe **175** is used with the adaptive phased array fast-acceleration gradient search algorithm, as disclosed in U.S. Pat. No. 5,810,888 to Fenn, to target the microwave radiation at the tumor site. The temperature sensed by the invasive temperature probe **175** in the tumor could be used as a real-time feedback signal during the treatment. This feedback signal **450** could be used to control the microwave output power level of the variable power amplifiers, which set and maintain the focal temperature at the tumor site in the range of 43 to 46° C. The power and phase delivered to the two channels of the phased array are adjusted adaptively using digital-to-analog converters under computer control.

Total Microwave Energy Dose can be used to estimate the required heating time. That is, Applicants realized that a non-interstitial equivalent temperature sensing means could replace the interstitial temperature probes, and that the Total Microwave Energy Dose reliably could be used to control the duration of treatment. The prostate compression, as mentioned earlier, reduces blood flow, which likely eliminates the effects of blood flow on the required microwave energy for treatment, and may reduce the variation in microwave energy that can be expected in microwave treatments.

Accordingly to a preferred embodiment, the total microwave energy delivered to the waveguide applicators to determine completion of the treatment is between 25 kilo Joules and 250 kilo Joules. The total amount of microwave energy dose that would destroy any cancerous or precancerous tissue would be approximately 175 kilo Joules. But, under certain conditions, the required microwave energy dose may be as low as 25 kilo Joules.

As applicants recognized, compression of a body that results in a smaller thickness may require less microwave energy dose (compared to a compression that results in a larger thickness) for effective treatments in preventing or destroying cancerous, pre-cancerous or benign lesions. It is important to select an appropriate initial microwave power level ($P_1$,$P_2$) delivered to each applicator as well as the proper microwave phase between the two applicators to focus the energy at the area to be treated.

During hyperthermia treatment, it is necessary to monitor the urethral and rectal wall temperatures so that they do not rise significantly above about 41 degrees Celsius for more than several minutes. The equivalent thermal dose for the urethral and rectal wall sensors can be calculated (Sapareto, et al., International Journal of Radiation Oncology Biology Physics, VoL 10, pp. 787-800, 1984) and can be used as a feedback signal. Typically, it is necessary to avoid delivering more than a few equivalent minutes thermal dose. Avoiding high urethral and rectal temperatures according to the invention is accomplished by adjusting the individual powers ($P_1$, $P_2$) delivered to the applicators during treatment either by manual or automatic computer control.

Doppler ultrasound can be used to measure blood flow in tumors and surrounding prostate tissue, before and during treatment to plan and adjust the microwave energy dose. For example, less energy dose is required when the tumor blood flow rate is reduced which can occur when the prostate is compressed and/or the tumor is heated to therapeutic temperatures. Alternatively, the water content and dielectric parameters of prostate tumor tissue from needle biopsies could be measured and used to determine, prior to the treatment, the required microwave energy dose. For example, higher water content and higher electrical conductivity in the tumor would reduce the amount of required microwave energy dose. In addition to the above variables, the size of the tumor impacts the required microwave energy dose. Larger tumors are more difficult to heat than smaller tumors and require a larger microwave energy dose. An initial treatment planning session involving a low-dose delivery of microwave energy to assess the heatability of the tumor, followed by a complete treatment at the full required microwave energy dose may be performed.

Simplified Microwave Radiation Theory

Microwave energy from hyperthermia applicators, in the near field of a body, radiates as a spherical wave with the electric-field amplitude varying, in part, as the inverse of the radial distance $r$ from the applicator. Additionally, the amplitude decays as an exponential function of the product of the attenuation constant $\alpha$ of the body tissue and the distance $d$ traversed (or depth) within the body as indicated in Figure 1. The electric-field phase varies linearly with distance according to the product of the phase propagation constant $\beta$ and distance d. For simplicity, dual-opposing applicators are analyzed here under the assumption that the applicator radiation is approximated by a plane wave. Mathematically, the plane-wave electric field versus depth in tissue is given by $E(d)=E_o \exp(-\alpha d) \exp(-i\beta d)$, where $E_o$ is the surface electric field (in general represented by an amplitude and phase angle), and $i$ is the imaginary number (Field and Hand, An Introduction to the Practical Aspects of Clinical Hyperthermia , Taylor & Francis, New York p. 263, 1990).

Plane-wave electromagnetic energy, at the microwave frequency of 915 MHz, attenuates at a rate of about 3 dB per cm in high-water content tissue, such as prostate tissue. Thus, a single radiating applicator has a significant fraction of its microwave energy absorbed by intervening superficial body tissue compared to the energy that irradiates deep tissue, likely creating a hot spot in superficial tissue. Since surface cooling with either air or water protects tissue only to a maximum depth of about 0.25 to 0.5 cm, in order to avoid hot spots, it is necessary to introduce a second phase-coherent applicator, having the same microwave radiation amplitude as the first applicator. The second phase-coherent applicator can theoretically increase the power (and hence the energy) delivered to deep tissue by a factor of four compared to a single applicator (Field and Hand, p. 290, 1990).

The phase characteristics of the electromagnetic radiation from two or more applicators (known as a phased array) can have a pronounced affect on the distribution of power delivered to different tissues. The relative specific absorption rate (SAR) in homogeneous tissue is approximated by the square of the electric-field amplitude $|E|^2$. The SAR is proportional to the rise in temperature over a given time interval. A simplified case, homogeneous prostate tissue, in which the microwave radiation is focused at a central tissue site, is described in detail below. As described in an article by Fenn et al., International Symposium on Electromagnetic Compatibility, Sendai, Japan, Vol. 10, No. 2, May 17-19, 1994, pp. 566-569, the effects of multiple microwave signal reflections within the phantom can be ignored.

The wavelength in homogeneous normal prostate tissue (with approximate dielectric constant 50 and electrical conductivity 1.3 S/m) is approximately 4.5 cm at 915 MHz, and the microwave loss is 3 dB/cm. The attenuation constant $\alpha$ is 0.34 radians/cm and the propagation constant $\beta$ is 1.4 radians/cm (or 80 degrees/cm). (For a compressed prostate thickness of 2.25 cm, the electric field of a single applicator radiating on the left side is $E_o$ at the surface of the prostate, $-i0.7E_o$ (where i represents a 90-degree phase shift) at the central position (1.125 cm deep), and $-0.5E_o$ at the right surface. Combining two phase coherent applicators yields an electric-field value of $0.5E_o$ on both surfaces and $-i1.4E_o$ at the central position (1.125 cm depth). Thus, for the compressed prostate, by squaring the above coherent E-fields to compute SAR, there is a significantly lower SAR at the surface, by about a factor of 2.0 compared to the central SAR. The 180-degree phase shift experienced by the microwave field transmitted through 2.25 cm of prostate tissue, partly cancels or nulls the field entering the tissue with 0-degree phase shift. Due to destructive interference of the microwaves away from the central focus lower temperatures in the superficial prostate tissues would be expected. Measurement and enforcement of lower SAR on the opposing surfaces effectively focuses the microwave energy deep in the prostate. In cases where it is desirable to radiate the superficial or peripheral zone prostate tissues more strongly, the compression thickness can be larger than 2.25 cm so that the propagating wave phase delay is longer and the two waves do not cancel at the surface, or only one of the transurethral or transrectal applicators (especially the transrectal applicator) can be used to heat the prostate.

Repeating the above calculation, but now for non-coherent applicators, for a compressed prostate thickness of 2.25 cm, the electric field of a single applicator radiating on the left side is $E_o$ at the surface of the prostate, $-i0.7E_o$ (where i represents a 90-degree phase shift) at the central position (1,125 cm deep), and $-0.5E_o$ at the light surface. Combining two applicators non-coherently, by squaring the individual E-fields and adding them, yields a SAR value of $1.5E_o^2$ on both surfaces and $0.98 E_o^2$ at the central position (1.125 cm depth). Thus, for the compressed prostate, by squaring the above non-coherent E-fields to compute SAR, there is a significantly higher SAR at the surface, by about a factor of 1.5 compared to the central SAR. For this reason, it is more difficult to heat deep prostate tissue with the non-coherent array compared to the coherent array. However, as mentioned earlier, for prostate cancer treatment some of the prostate cancer cells can be located close to the rectum and non-coherent treatment may provide adequate heating.

The adaptive phased array system according to the preferred embodiment of the invention uses two microwave channels, fed by a common oscillator 105, containing two electronically adjustable phase shifters 120 to focus the microwave energy at an E-field feedback probe 175. This embodiment of the inventive adaptive phased array system has significant advantage over a non-adaptive phased array. A non-adaptive phased array with two channels could, in theory, produce a null, a maximum, or an intermediate value of E-field depending on whether the two waves are 180 degrees out-of phase, completely in-phase, or partly out-of phase, respectively. That is, the microwave phase delivered to the microwave applicators, according to the preferred embodiment of the invention, can be adjusted between -180 degrees and 180 degrees before and during the treatment to create a focused field in the prostate tissue.

The adaptive phased array according to the preferred embodiment of the invention automatically focuses the E-field in the presence of all scattering structures in the tissue. Thus, the adaptive phased array according to the preferred embodiment of the invention should provide more reliable deep focused heating compared to manually adjusted or pre-treatment planning controlled phased arrays as described in U.S. Patent No. 4,589,423 to Turner. Furthermore, the adaptive phased array system according to the preferred embodiment of the invention does not use an invasive temperature probe, which could scatter or alter the E-field at the tumor site.

Calculation of Microwave Energy

Electrical energy consumption is commonly expressed in units of kilowatt hours. Mathematically, the expression for the microwave energy $W$ delivered by an applicator is given by (Vitrogan, Elements of Electric and Magnetic Circuits, Rinehart Press, San Francisco, pp. 31-34, 1971):

$$W = \Delta t \Sigma P_i. \quad (1)$$

In the above equation, $\Delta t$ represents the constant intervals (in seconds) in which microwave power is measured and the summation $\Sigma$ is over the complete treatment interval with the power (in Watts) in the $i$th interval denoted by $P_L$

The microwave energy $W$ has units of watt-seconds, which is also designated as Joules. For example, in three consecutive 60-second intervals if the microwave power is 30 watts, 50 watts, 60 watts, respectively, the total microwave energy delivered in 180 seconds is calculated as $W$ = 60 (30 + 50 + 60) = 8,400 watt-seconds = 8,400 Joules = 8.4 kJ.

To understand better the focused energy per unit time $W'$ (where ' denotes prime) deposited at a central position in homogeneous prostate tissue of varying thickness (denoted by $D$) by dual-opposing applicators, consider the following calculation for coherent treatments. Let $P_1$ and $P_2$ be the power delivered to the two applicators, respectively. The electric field radiated by each applicator is proportional to the square root of the power delivered to the applicator. Assuming symmetry, the radiated fields are in-phase at the central focused position from the two applicators. Assuming equal power from each applicator, that is, $P_1 = P_2 = P$, and plane wave illumination, then the focused energy per unit time at the central depth is expressed as

$$W'(D) = |E|^2 = 4P \exp(-\alpha D). \quad (2)$$

Calculation of Equivalent Thermal Dose

The cumulative or total equivalent thermal dose relative to 43 degrees Celsius is calculated as a summation (Sapareto, et al., International Journal of Radiation Oncology Biology Physics, Vol. 10, pp. 787-800.1984):

$$t_{43°C} \text{ equivalent minutes } = \Delta t \Sigma R^{(43-T)}, \quad (3)$$

where E is the summation over a series of temperature measurements during the treatment, $T$ is the series of temperature measurements ($T_1$, $T_2$, $T_3$, ...), $\Delta t$ is the constant interval of time (units of seconds and converted to minutes) between measurements, $R$ is equal to 0.5 if $T>43°$ C and $R$ is equal to 0.25 if $T<43°$ C. The equivalent thermal dose calculation is useful for assessing any possible heat damage to the prostate tissues, urethra and rectum.

**Equivalents**

Although the hyperthermia system described herein is with respect to the treatment of prostate carcinomas and benign prostate lesions, the invention is applicable to the treatment of other types of cancers such as breast, liver, lung, and ovarian. It is also understood that larger or smaller numbers of array antenna applicators, or single antenna applicators such as transurethral or transrectal, may be used with similar results. Some of the methods and techniques described herein are also applicable to ultrasound hyperthermia system particularly the use of energy dose for feedback control. The system according to the invention can be used to enhance radiation therapy or for targeted drug delivery and/or targeted gene delivery using thermosensitive liposomes or for targeted gene therapy. The invention is also applicable to non-medical hyperthermia systems, such as those used for industrial heating.

**Claims**

1. A system for treating of one of cancerous, pre-cancerous, pre-benign and benign conditions of a prostate by irradiation of the prostate with concentrated energy, the system comprising at least one energy applicator (110, 111) for irradiating the prostate with energy, means for setting the initial energy power delivered to said at least one energy applicator, means (410) for monitoring temperatures of walls of the urethra and rectum adjacent the prostate and means for adjusting the relative energy power delivered to said at least one applicator (110, 111) during treatment based on the monitored urethral and rectal wall temperatures, **characterized by**

   a) means for monitoring the energy delivered to said at least one energy applicator; and
   b) means for automatically terminating the treatment when a desired total energy dose has been delivered by said at least one energy applicator to the prostate.

2. The system according to claim 1, wherein the concentrated energy is microwave energy and further comprising:

   an E-field probe (175) for insertion to an appropriate depth in tissue of the prostate; and
   means for setting the initial relative microwave phase delivered to each applicator to focus the microwave energy at the E-field probe (175) positioned in the prostate tissue.

3. The system according to claim 2, further comprising a system providing ultrasound guidance during insertion of the E-probe (175) into the central depth of the prostate tissue or in a prostate lesion.

4. The system according to claim 1, wherein the means (410) for monitoring the urethral and rectal wall temperatures include temperature probes (410) adapted to be brought in pressure contact with the walls of the urethra and rectum.

5. The system according to claim 1, wherein said at least one energy applicator (110, 111) is adapted to be oriented in at least one of the urethra and rectum and further comprising means for compressing the prostate and for fixing positions of said at least one energy applicator.

6. The system according to claim 5, wherein the means for compressing the prostate and for fixing positions of the applicators includes a balloon (112, 113) that is inflated with one of distilled and deionized water, said balloon (112, 113) is adapted for being inserted in at least one of the urethra and rectum.

7. The system according to claim 1, wherein the concentrated energy is one of microwave, ultrasound, radiofrequency, and laser energy and wherein one or more coherent or non-coherent energy applicators are adapted to surround the prostate to selectively irradiate the prostate tissue with energy to treat one of cancerous, pre-cancerous, benign and pre-benign conditions of the prostate.

8. The system according to claim 1, wherein the concentrated energy is one of microwave, ultrasound, radiofrequency, and laser energy and wherein said at least one energy applicator is adapted to be inserted interstitially within the prostate to selectively irradiate the prostate tissue with concentrated energy to treat cancerous, pre-cancerous, and benign conditions of the prostate.

9. The system according to claim 1, wherein the concentrated energy is microwave energy and said at least one energy applicator is adapted to be oriented in at least one of the urethra and rectum, and further comprising means for cooling the microwave applicators in the urethra and rectum with water, and means for adjusting the water cooling temperature for the transurethral and transrectal applicators to protect the urethra and rectum form overheating.

10. The system according to claim 1, wherein the concentrated energy is microwave energy and further comprising a fluid-filled catheter (300, 301) wherein said at least one microwave applicator is disposed in the fluid-filled catheter (300, 301) and the fluid-filled catheter (300, 301) couples microwave energy from said at least one applicator (110, 111) to the tissue to be treated.

11. The system according to claim 1, wherein the concentrated energy is microwave energy and the frequency of the microwave energy is between 100 MHz and 10 GHz.

12. The system according to claim 1, wherein the concentrated energy is microwave energy, said system comprising two microwave applicators adapted to be oriented in the urethra and rectum, respectively; and further comprising means for adjusting the relative microwave phase delivered to the two microwave applicators, the relative microwave phase being adjusted between - 180 degrees and 180 degrees before and during the treatment to create a focused field in the prostate tissue.

13. The system according to claim 1, wherein the concentrated energy is microwave energy and the initial microwave power delivered to said at least one applicator is between 0 Watts and 70 Watts.

14. The system according to claim 1, wherein the concentrated energy is microwave energy and the means for adjusting the microwave power delivered to said at least one applicator (110, 111) adjusts the microwave power over the range of 0 to 150 Watts during the treatment to deliver the desired microwave energy dose and to avoid overheating the healthy tissue and walls surrounding the area to be treated.

15. The system according to claim 1, wherein the concentrated energy is microwave energy and further comprising means for determining the total microwave energy delivered to said at least one microwave applicator (110, 111) and means (260) for displaying the total microwave energy in real time during the treatment.

16. The system according to claim 1, wherein the concentrated energy is microwave energy and the total microwave energy delivered to said at least one applicator (110, 111) for the complete treatment is between 25 kilo Joules and 250 kilo Joules.

17. The system according to claim 2, wherein the means (410) for monitoring temperatures of the urethral and rectal walls is a temperature probe (176) that is adapted to be inserted at an appropriate depth in the prostate tissue.

18. The system according to claim 1, wherein the concentrated energy is microwave energy and the total microwave energy dose produces a total equivalent thermal dose in prostate tumors which is approximately between 60 minutes and 400 minutes relative to 43 degrees Celsius.

19. The system according to claim 2, further comprising means for monitoring the microwave power level delivered to the E-field probe (175), wherein the total microwave energy received by the E-field probe (175) is used as feedback to determine the length of treatment.

20. The system of claim 4, further comprising second means (176) for monitoring the urethral and rectal wall temperatures, said second temperature monitoring means (176) being a probe adapted to be inserted into the urethra or the rectum.

21. The system of claim 1, wherein the concentrated energy is microwave energy and further comprising:

two non-interstitial E-fields sensors adapted to be positioned on urethral and rectal wall surfaces adjacent the prostate; and means for setting and adjusting the initial relative energy phase delivered to said at least one applicator to minimize the energy at the E-field sensors positioned on urethal and rectal wall surfaces adjacent the prostate to focus the energy at the prostate tissue to be treated.

**Patentansprüche**

1. Ein System zur Behandlung eines kanzerösen, präkanzerösen, prä-benignen oder gutartigen Zustands einer Prostata durch Bestrahlung der Prostata mit konzentrierter Energie, wobei das System zumindest einen Energieapplikator (110, 111) zum Bestrahlen der Prostata mit Energie, Mittel zum Einstellen der anfänglichen Energieleistung, die an den zumindest einen Energieapplikator geliefert wird, Mittel (410) zum Überwachen von Temperaturen von Wänden der Urethra und des Rektums, die der Prostata benachbart sind, und Mittel zum Regulieren der relativen Energieleistung, die an den genannten zumindest einen Energieapplikator (110, 111) geliefert wird, während der Behandlung auf der Grundlage der überwachten Temperaturen der urethralen und rektalen Wände umfasst, **gekennzeichnet durch**

a) Mittel zum Überwachen der Energie, die an den genannten zumindest einen Energieapplikator geliefert wird; und

b) Mittel zum automatischen Beenden der Behandlung, wenn eine gewünschte Gesamtenergiedosis von dem zumindest einen Energieapplikator an die Prostata geliefert worden ist.

2. Das System gemäß Anspruch 1, worin die konzentrierte Energie Mikrowellenenergie ist, und weiterhin umfassend:

eine E-Feld-Sonde (175) zum Einführen in einer geeigneten Tiefe in das Gewebe der Prostata; und
Mittel zum Einstellen der anfänglichen Mikrowellenphase, die an jeden Applikator geliefert wird, um die Mikrowellenenergie an der E-Feld-Sonde (175), die in dem Prostatagewebe positioniert ist, zu fokussieren.

3. Das System gemäß Anspruch 2, weiterhin ein System umfassend, das eine Ultraschallführung während der Einführung der E-Feld-Sonde (175) in die zentrale Tiefe des Prostatagewebes oder in eine Prostataläsion zur Verfügung stellt.

4. Das System gemäß Anspruch 1, worin die Mittel (410) zum Überwachen von Temperaturen der urethralen und rektalen Wände Temperatursonden (410) einschließen, die geeignet sind, in Druckkontakt mit den Wänden der Urethra und des Rektums gebracht zu werden.

5. Das System gemäß Anspruch 1, worin der genannte zumindest eine Energieapplikator (110, 111) geeignet ist, in zumindest entweder der Urethra oder dem Rektum ausgerichtet zu werden, und weiterhin ein Mittel zum Zusammendrücken der Prostata und zum Fixieren von Positionen des genannten zumindest einen Energieapplikators (110, 111) umfassend.

6. Das System gemäß Anspruch 5, worin das Mittel zum Zusammendrücken der Prostata und zum Fixieren von Positionen des Applikators einen Ballon (112, 113) einschließt, der entweder mit destilliertem oder deionisiertem Wasser aufgepumpt ist, wobei der genannte Ballon (112, 113) dazu geeignet ist, in zumindest entweder die Urethra oder das Rektum eingeführt zu werden.

7. Das System gemäß Anspruch 1, worin die konzentrierte Energie Mikrowellen-, Ultraschall-, Hochfrequenz- oder Laserenergie ist, und worin einer oder mehrere kohärente oder nicht-kohärente Energieapplikatoren geeignet sind, die Prostata zu umgeben, um selektiv das Prostatagewebe mit Energie zu bestrahlen, um einen kanzerösen, präkanzerösen, gutartigen oder prä-benignen Zustand der Prostata zu behandeln.

8. Das System gemäß Anspruch 1, worin die konzentrierte Energie Mikrowellen-, Ultraschall-, Hochfrequenz- oder Laserenergie ist, und worin der genannte zumindest eine Energieapplikator geeignet ist, interstitiell in die Prostata eingeführt zu werden, um selektiv das Prostatagewebe mit konzentrierter Energie zu bestrahlen, um kanzeröse, präkanzeröse und gutartige Zustände der Prostata zu behandeln.

9. Das System gemäß Anspruch 1, worin die konzentrierte Energie Mikrowellenenergie ist und der genannte zumindest eine Energieapplikator geeignet ist, in zumindest der Urethra oder dem Rektum ausgerichtet zu werden, und weiterhin Mittel zum Kühlen der Mikrowellenapplikatoren in der Urethra und dem Rektum mit Wasser und Mittel zum Regulieren der Kühlwassertemperatur für die urethralen und rektalen Applikatoren umfassend, um die Urethra und das Rektum vor einem Überhitzen zu schützen.

10. Das System gemäß Anspruch 1, worin die konzentrierte Energie Mikrowellenenergie ist und weiterhin einen fluidgefüllten Katheter (300, 301) umfassend, wobei der genannte zumindest eine Mikrowellenapplikator in dem fluidgefüllten Katheter (300, 301) angeordnet ist, und der fluidgefüllte Katheter (300, 301) Mikrowellenenergie von dem genannten zumindest einen Energieapplikator (110, 111) in das zu behandelnde Gewebe einkoppelt.

**11.** Das System gemäß Anspruch 1, worin die konzentrierte Energie Mikrowellenenergie ist und die Frequenz der Mikrowellenenergie zwischen 100 MHz und 10 GHz liegt.

**12.** Das System gemäß Anspruch 1, worin die konzentrierte Energie Mikrowellenenergie ist, wobei das genannte System zwei Mikrowellenapplikatoren umfasst, die geeignet sind, in der Urethra bzw. dem Rektum ausgerichtet zu werden; und weiterhin Mittel zum Regulieren der relativen Mikrowellenphase, die an die zwei Mikrowellenapplikatoren geliefert wird, umfassend, wobei vor und während der Behandlung die relative Mikrowellenphase zwischen -180 Grad und 180 Grad reguliert wird, um ein fokussiertes Feld in dem Prostatagewebe zu erzeugen.

**13.** Das System gemäß Anspruch 1, worin die konzentrierte Energie Mikrowellenenergie ist, und die anfängliche Mikrowellenleistung, die an den genannten zumindest einen Mikrowellenapplikator geliefert wird, zwischen 0 Watt und 70 Watt beträgt.

**14.** Das System gemäß Anspruch 1, worin die konzentrierte Energie Mikrowellenenergie ist, und das Mittel zum Regulieren der Mikrowellenleistung, die an den genannten zumindest einen Mikrowellenapplikator (110, 111) geliefert wird, die Mikrowellenleistung während der Behandlung über den Bereich von 0 bis 150 Watt reguliert, um die gewünschte Mikrowellenenergiedosis zu liefern und ein Überhitzen des gesunden Gewebes und der Wände, die den zu behandelnden Bereich umgeben, zu vermeiden.

**15.** Das System gemäß Anspruch 1, worin die konzentrierte Energie Mikrowellenenergie ist und weiterhin Mittel zum Bestimmen der Gesamtmikrowellenenergie, die an den genannten zumindest einen Mikrowellenapplikator (110, 111) geliefert wird, und Mittel (260) zum Anzeigen der Gesamtmikrowellenenergie während der Behandlung in Echtzeit umfassend.

**16.** Das System gemäß Anspruch 1, worin die konzentrierte Energie Mikrowellenenergie ist und die Gesamtmikrowellenenergie, die an den genannten zumindest einen Applikator (110, 111) für die gesamte Behandlung geliefert wird, zwischen 25 Kilojoules und 250 Kilojoule beträgt.

**17.** Das System gemäß Anspruch 2, worin das Mittel (410) zum Überwachen der Temperaturen der urethralen und rektalen Wände eine Temperatursonde (176) ist, die geeignet ist, in einer geeigneten Tiefe in das Prostatagewebe eingeführt zu werden.

**18.** Das System gemäß Anspruch 1, worin die konzentrierte Energie Mikrowellenenergie ist und die Gesamtmikrowellenenergiedosis eine thermische Gesamtäquivalenzdosis in Prostatatumoren erzeugt, die ungefähr zwischen 60 Minuten und 400 Minuten in Bezug auf 43 Grad Celsius beträgt.

**19.** Das System gemäß Anspruch 2, weiterhin Mittel zum Überwachen des Mikrowellenleistungsniveaus, das an die E-Feld-Sonde (175) geliefert wird, umfassend, worin die Gesamtmikrowellenenergie, die von der E-Feld-Sonde (175) empfangen wird, als Feedback verwendet wird, um die Länge der Behandlung zu bestimmen.

**20.** Das System von Anspruch 4, weiterhin zweite Mittel (176) zum Überwachen der urethralen und rektalen Wandtemperaturen umfassend, wobei das genannte zweite Temperaturüberwachungsmittel (176) eine Sonde ist, die dazu geeignet ist, in die Urethra oder das Rektum eingeführt zu werden.

**21.** Das System gemäß Anspruch 1, worin die konzentrierte Energie Mikrowellenenergie ist, und weiterhin umfassend:

zwei nicht-interstitielle E-Feld-Sensoren, die geeignet sind, auf den der Prostata benachbarten urethralen und rektalen Wandoberflächen positioniert zu werden; und Mittel zum Einstellen und Regulieren der anfänglichen relativen Energiephase, die an den genannten zumindest einen Applikator geliefert wird, um die Energie an den E-Feld-Sensoren, die auf den der Prostata benachbarten urethralen und rektalen Wandoberflächen positioniert sind, zu minimieren, um die Energie in dem zu behandelnden Prostatagewebe zu fokussieren.

**Revendications**

**1.** Système pour traiter un état, choisi parmi les états cancéreux, pré-cancéreux, pré-bénins et bénins, d'une prostate, par irradiation de la prostate avec une énergie concentrée, le système comprenant au moins un applicateur d'énergie (110, 111) pour irradier la prostate avec une énergie, des moyens pour régler la puissance énergétique initiale délivrée audit applicateur d'énergie, des moyens (410) pour surveiller les températures des parois de l'urètre et du rectum adjacentes à la prostate et des moyens pour ajuster la puissance énergétique relative délivrée audit applicateur (110, 111) durant le traitement sur la base des températures surveillées des parois urétrale et rectale, **caractérisé par**

    a) des moyens pour surveiller l'énergie délivrée audit applicateur d'énergie ; et
    b) des moyens pour terminer automatiquement le traitement quand une dose énergétique totale souhaitée a été délivrée à la prostate par ledit applicateur d'énergie.

2. Système selon la revendication 1, dans lequel l'énergie concentrée est une énergie de micro-ondes, et qui comprend en outre :

    une sonde de champ E (175) à insérer à une profondeur appropriée dans le tissu de la prostate ; et
    des moyens pour régler la phase de micro-ondes relative initiale délivrée à chaque applicateur pour focaliser l'énergie de micro-ondes au niveau de la sonde de champ E (175) positionnée dans le tissu de la prostate.

3. Système selon la revendication 2, comprenant en outre un système permettant un guidage par ultrasons durant l'insertion de la sonde E (175) dans la profondeur centrale du tissu de la prostate ou dans une lésion de la prostate.

4. Système selon la revendication 1, dans lequel les moyens (410) pour surveiller les températures des parois urétrale et rectale comprennent des sondes de température (410) adaptées pour être amenées en contact par pression avec les parois de l'urètre et du rectum.

5. Système selon la revendication 1, dans lequel ledit applicateur d'énergie (110, 111) est adapté pour être orienté dans au moins l'un parmi l'urètre et le rectum, et qui comprend en outre des moyens pour comprimer la prostate et pour fixer des positions dudit applicateur d'énergie.

6. Système selon la revendication 5, dans lequel les moyens pour comprimer la prostate et pour fixer les positions des applicateurs comprennent un ballonnet (112, 113) qui est gonflé avec l'une parmi l'eau distillée et l'eau désionisée, ledit ballonnet (112, 113) étant adapté pour être inséré dans au moins l'un parmi l'urètre et le rectum.

7. Système selon la revendication 1, dans lequel l'énergie concentrée est l'une parmi les énergies de micro-ondes, d'ultrasons, de radiofréquences, et de laser, et dans lequel un ou plusieurs applicateurs d'énergie cohérente ou non-cohérente sont adaptés pour entourer la prostate de façon à irradier sélectivement le tissu de la prostate avec de l'énergie pour traiter un état parmi les états cancéreux, pré-cancéreux, bénins et pré-bénins de la prostate.

8. Système selon la revendication 1, dans lequel l'énergie concentrée est l'une parmi les énergies de micro-ondes, d'ultrasons, de radiofréquences, et de laser, et dans lequel ledit applicateur d'énergie est adapté pour être inséré de manière interstitielle à l'intérieur de la prostate pour irradier sélectivement le tissu de la prostate avec de l'énergie concentrée pour traiter des états cancéreux, pré-cancéreux et bénins de la prostate.

9. Système selon la revendication 1, dans lequel l'énergie concentrée est une énergie de micro-ondes et ledit applicateur d'énergie est adapté pour être orienté dans au moins l'un parmi l'urètre et le rectum, et qui comprend en outre des moyens pour refroidir les applicateurs de micro-ondes dans l'urètre et le rectum avec de l'eau ; et des moyens pour ajuster la température de refroidissement par eau pour les applicateurs trans-urétraux et trans-rectaux afin de protéger l'urètre et le rectum vis-à-vis d'un chauffage excessif.

10. Système selon la revendication 1, dans lequel l'énergie concentrée est une énergie de micro-ondes, et qui comprend en outre un cathéter rempli de fluide (300, 301), dans lequel ledit applicateur de micro-ondes est disposé dans le cathéter rempli de fluide (300, 301) et le cathéter rempli de fluide (300, 301) couple l'énergie de micro-ondes provenant dudit applicateur (110, 111) avec le tissu devant être traité.

11. Système selon la revendication 1, dans lequel l'énergie concentrée est une énergie de micro-ondes et la fréquence de l'énergie de micro-ondes est comprise entre 100 MHz et 10 GHz.

12. Système selon la revendication 1, dans lequel l'énergie concentrée est une énergie de micro-ondes, ledit système comprenant deux applicateurs de micro-ondes adaptés pour être orientés dans l'urètre et le rectum, respectivement ; et comprenant en outre des moyens pour ajuster la phase de micro-ondes relative délivrée aux deux applicateurs de micro-ondes, la phase de micro-ondes relative étant ajustée entre -180 degrés et 180 degrés avant et pendant le traitement pour créer un champ focalisé dans le tissu de la prostate.

13. Système selon la revendication 1, dans lequel l'énergie concentrée est une énergie de micro-ondes et la puissance initiale de micro-ondes délivrée audit applicateur est comprise entre 0 watt et 70 watts.

14. Système selon la revendication 1, dans lequel l'énergie concentrée est une énergie de micro-ondes et les moyens pour ajuster la puissance de micro-ondes délivrée audit applicateur (110, 111) ajuste la puissance de micro-ondes sur la plage de 0 à 150 watts durant le traitement pour délivrer la dose d'énergie de micro-ondes souhaitée et pour éviter un chauffage excessif du tissu sain et des parois entourant la zone devant être traitée.

15. Système selon la revendication 1, dans lequel l'énergie concentrée est une énergie de micro-ondes, et qui comprend en outre des moyens pour déterminer l'énergie de micro-ondes totale délivrée audit applicateur de micro-ondes (110, 111) et des moyens (260) pour afficher l'énergie de micro-ondes totale en temps réel durant le traitement.

16. Système selon la revendication 1, dans lequel l'énergie concentrée est une énergie de micro-ondes et l'énergie de micro-ondes totale délivrée audit applicateur (110, 111) pour le traitement complet est compris entre 25 kilojoules et 250 kilojoules.

17. Système selon la revendication 2, dans lequel les moyens (410) pour surveiller les températures des parois urétrale et rectale est une sonde de température (176) qui est adaptée pour être insérée à une profondeur appropriée dans le tissu de la prostate.

18. Système selon la revendication 1, dans lequel l'énergie concentrée est une énergie de micro-ondes et la dose d'énergie de micro-ondes totale produit une dose thermique équivalente totale dans les tumeurs de la prostate qui est comprise entre environ 60 minutes et 400 minutes pour 43 degrés Celsius.

19. Système selon la revendication 2, comprenant en outre des moyens pour surveiller le niveau de puissance de micro-ondes délivré à la sonde de champ E (175), l'énergie de micro-ondes totale reçue par la sonde de champ E (175) étant utilisée en tant qu'information en retour pour déterminer la durée du traitement.

20. Système selon la revendication 4, comprenant en outre des seconds moyens (176) pour surveiller les températures des parois urétrale et rectale, lesdits seconds moyens de surveillance de la température (176) étant constitués d'une sonde adaptée pour être insérée dans l'urètre ou le rectum.

21. Système selon la revendication 1, dans lequel l'énergie concentrée est une énergie de micro-ondes, et qui comprend en outre :

    deux capteurs de champ E non-interstitiels adaptés pour être positionnés sur les surfaces des parois urétrale et rectale adjacentes à la prostate ; et des moyens pour régler et ajuster la phase d'énergie relative initiale délivrée audit applicateur pour minimiser l'énergie au niveau des capteurs de champ E positionnés sur les surfaces des parois urétrale et rectale adjacentes à la prostate de manière à focaliser l'énergie au niveau du tissu de la prostate devant être traité.

FIG. 1

FIG. 2